Europäisches Patentamt

European Patent Office

Office européen des brevets

(11) Veröffentlichungsnummer: **0 416 220 A1**

# (12) EUROPÄISCHE PATENTANMELDUNG

(21) Anmeldenummer: 90110661.7

(22) Anmeldetag: 06.06.90

(51) Int. Cl.⁵: **C07D 241/26**

(30) Priorität: 04.08.89 DE 3925847

(43) Veröffentlichungstag der Anmeldung:
13.03.91 Patentblatt 91/11

(84) Benannte Vertragsstaaten:
**CH DE ES FR GB IT LI**

(71) Anmelder: **HÜLS AKTIENGESELLSCHAFT**
**Patentabteilung / PB 15 - Postfach 13 20**
**W-4370 Marl 1(DE)**

(72) Erfinder: **Feld, Marcel, Dr.**
**Im Lochgarten 56**
**W-5000 Köln 90(DE)**

(54) Verfahren zur Herstellung von 3-Aminopyrazin-2-carbonsäuremethylester.

(57) 3-Aminopyrazin-2-carbonsäuremethylester wird aus Alkalisalzen der 3-Aminopyrazin-2-carbonsäure durch Umsetzung mit Methylbromid in Dimethylformamid und/oder Dimethylacetamid hergestellt.

EP 0 416 220 A1

# VERFAHREN ZUR HERSTELLUNG VON 3-AMINOPYRAZIN-2-CARBONSÄUREMETHYLESTER

Die Erfindung betrifft ein Verfahren zur Herstellung von 3-Aminopyrazin-2-carbonsäuremethylester, der als Zwischenprodukt insbesondere für die Synthese von PharmaWirkstoffen, beispielsweise für das Diureticum Amilorid Bedeutung hat.

Nach bekannten Verfahren wird das Produkt durch säurekatalysierte Veresterung von 3-Aminopyrazin-2-carbonsäure mit Methanol gewonnen. Die für diese Veresterung genannten Ausbeuten von ca. 50% (LIEBIG's Ann. 660 (1962), 98, 101) bzw. 72% Rohprodukte (J.Am. Chem. Soc. 67 (1945), 1712) sind sehr unbefriedigend.

Es bestand daher die Aufgabe, 3-Aminopyrazin-2-carbonsäuremethylester hoher Reinheit in befriedigender Ausbeute herzustellen.

Gegenstand der Erfindung ist das Verfahren nach dem Patentanspruch. Demnach werden Alkalisalze der 3-Aminopyrazin-2-carbonsäure in Dimethylformamid und/oder Dimethylacetamid als Reaktionsmedium unter milden Bedingungen mit Methylbromid selektiv zum 3-Aminopyrazin-2-carbonsäuremethylester umgesetzt.

Das als Ausgangsstoff für das erfindungsgemäße Verfahren benötigte Alkalisalz der 3-Aminopyrazin-2-carbonsäure ist durch alkalische Hydrolyse von Lumazin zugänglich (J. Amer.Chem. Soc. 67 (1945), 802). Sofern dabei die Säure isoliert wird, kann daraus wieder das benötigte Alkalisalz hergestellt werden. Es ist auch möglich, beim erfindungsgemäßen Verfahren die 3-Aminopyrazin-2-carbonsäure zusammmen mit einer geeigneten Alkalikomponente, vorzugsweise einem Alkalialkoholat, einzusetzen. Bevorzugt wird beim erfindungsgemäßen Verfahren das Kaliumsalz der 3 - aminopyrazin-2-carbonsäure als Ausgangsprodukt verwendet. Das Na-Salz ist ebenfalls verwendbar.

Zur Unterdrückung unerwünschter Neben-und Folgereaktionen, insbesondere der Methylierung der Aminogruppe von Ausgangsstoff oder Produkt, erfolgt die erfindungsgemäße Umsetzung bei milden Temperaturen zwischen 20 und 80° C, vorzugsweise unter 60° C und insbesondere im Temperaturbereich von 30 bis 50° C. Unter diesen Bedingungen gelingt die Methylierung des Alkalisalzes der 3-Aminopyrazin-2-carbonsäure zum Produkt in Dimethylformamid oder Dimethylacetamid mit überraschend hoher Selektivität.

Als Reaktionsmedium werden demnach erfindungsgemäß Dimethylformamid, Dimethylacetamid oder beliebige Mischungen dieser beiden Lösemittel eingesetzt. Das Reaktionsmedium kann neben den genannten Amiden als Hauptbestandteile zusätzlilch geringe Mengen anderer Lösemittel enthalten. Dies kann sich beispielsweise dann ergeben, wenn als Ausgangsprodukt die 3-Aminopyrazin-2-carbonsäure zusammen mit einer Alkalikomponente zum Einsatz kommt. Mischungen von Dimethylformamid oder Dimethylacetamid mit größeren Mengen eines Alkohols haben sich für das erfindungsgemäße Verfahren allerdings als ungeeignet erwiesen. Nachteilig sind ferner bereits kleine Mengen Wasser. Das Dimethylformamid und/oder Dimethylacetamid kommt dementsprechend wasserarm mit einem Wassergehalt von unter 5, vorzugsweise von unter 2 Gew.-%, zum Einsatz.

Die Menge des Reaktionsmediums muß ausreichen, um mit dem eingesetzten Alkalisalz bzw. den Reaktionsprodukten 3-Aminopyrazin-2-carbonsäuremethylester und Alkalibromid eine rühr- bzw. pumpbare Mischung zu erhalten. Diese Bedingung wird von 1 bis 1,5 Gew.-Teilen Dimethylformamid und/oder Dimethylacetamid pro Gew.-Teil Alkalisalz der 3-Aminopyrazin-2-carbonsäure erfüllt. Somit werden beim erfindungsgemäßen Verfahren pro Gew.-Teil des Alkalisalzes 1 bis 6, vorzugsweise 1 bis 3 Gew.-Teile Dimethylformamid und/oder Dimethylacetamid, eingesetzt. Als zusätzliche Lösungsmittelkomponente kommt insbesondere noch Acetonitril in Betracht.

Das Methylbromid wird beim erfindungsgemäßen Verfahren gegenüber dem Alkalisalz der 3-Aminopyrazin-2-carbonsäure vorzugsweise im Überschuß von 10 bis 200 Mol% eingesetzt. Es kann ganz oder teilweise im Reaktionsmedium gelöst vorgelegt, oder in eine vorgelegte Suspension des Alkalisalzes in Dimethylformamid und/oder Dimethylacetamid gasförmig oder flüssig, ggf. erst unter Reaktionsbedingungen und über einen längeren Zeitraum von mehreren Stunden, eingeleitet werden. Die Zugabe des Methylbromids kann auch in Form einer Lösung erfolgen.

Der 3-Aminopyrazin-2-carbonsäuremethylester fällt zusammen mit Alkalibromid größtenteils ungelöst in fester Form an und kann durch eine übliche Fest-Flüssig-Trennung aus dem Reaktionsgemisch abgetrennt werden. Die Abtrennung des Alkalibromids erfolgt dann durch gründliche Wäsche mit Wasser bzw. durch Umkristallisation aus Wasser. Zur Aufbereitung kann das Reaktionsgemisch allerdings auch mit Wasser verdünnt und das Zielprodukt durch eine Fest-Flüssig-Trennung isoliert werden.

Beispiel 1

70,8 g Kaliumsalz der 3-Aminopyrazin-2-carbonsäure (0,4 Mol) werden unter Rühren in eine Lösung von 35,5 g Methylbromid (0,37 Mol) in 80 g Dimethylformamid eingetragen. In die auf 43° C erwärmte Suspension Wurden dann innerhalb von 6 h weitere 38,5 g (0,4 Mol) Methylbromid eingeleitet. Nach weiteren 15 h bei Raumtemperatur wurde die Suspension mit 150g Wasser verdünnt, filtriert, der Filterkuchen mit Wasser gewaschen und getrocknet. Es wurden 46,6 g (79,4% d.Th.) 3-Aminopyrazin-2-carbonsäuremethylester mit einer Reinheit von 99,5% erhalten.

Beispiel 2

Analog Beispiel 1 wurden 70,8 g des Kaliumsalzes in eine Lösung von 47,1 g $CH_3Br$ (0,5 Mol) in 85 g Dimethylacetamid eingetragen und die Suspension 5 h bei 43 bis 48° C gerührt. Die Aufarbeitung ergab dann 53,2 g (86,9% d.Th.) 3-Aminopyrazin-2-carbonsäure-methylester gleicher Qualität wie in Beispiel 1.

**Ansprüche**

1. Verfahren zur Herstellung von 3-Aminopyrazin-2-carbonsäuremethylester, dadurch gekennzeichnet, daß ein Alkalisalz der 3-Aminopyrazin-2-carbonsäure in einem aus Dimethylformamid und/oder Dimethylacetamid als Hauptkomponenten bestehenden Reaktionsmedium mit Methylbromid umgesetzt wird.
2. Verfahren nach Anspruch 1, dadurch gekennzeichnet, daß die Umsetzung bei Temperaturen zwischen 20 und 80° C, vorzugsweise unter 60° C und besonders bevorzugt im Temperaturbereich von 30 bis 50° C durchgeführt wird.
3. Verfahren nach Anspruch 1 oder 2, dadurch gekennzeichnet, daß das Kaliumsalz der 3-Aminopyrazin-2-carbonsäure eingesetzt wird.
4. Verfahren nach einem der Ansprüche 1 bis 3, dadurch gekennzeichnet, daß pro Mol Alkalisalz der 3-Aminopyrazin-2-carbonsäure 1,1 bis 3 Mol Methylbromid eingesetzt werden.
5. Verfahren nach einem der Ansprüche 1 bis 4, dadurch gekennzeichnet, daß pro Gew.-Teil Alkalisalz der 3-Aminopyrazin-2-carbonsäure 1 bis 6, vorzugsweise 1 bis 3 Gew.-Teile Dimethylformamid und/oder Dimethylacetamid verwendet werden.
6. Verfahren nach einem der Ansprüche 1 bis 5, dadurch gekennzeichnet, daß das Dimethylformamid und/oder Dimethylacetamid als Hauptkomponenten enthaltende Reaktionsmedium einen Wassergehalt von weniger als 5, vorzugsweise von weniger als 2 Gew.-% enthält.

**Europäisches Patentamt**

# EUROPÄISCHER RECHERCHENBERICHT

Nummer der Anmeldung

## EP 90 11 0661

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-3 418 360 (J.G.D. SCHULZ et al.)<br>* ganzes Dokument * | 1-6 | C 07 D 241/26 |
| A | US-A-3 988 341 (W.S. SAARI et al.)<br>* Ansprüche 1,2,5; Spalte 2, Zeilen 40-52 * | 1,2 | |
| A | US-A-3 461 156 (W.L. FIERCE)<br>* Anspruch 1 * | 1,2 | |
| A,D | JOURNAL OF THE AMERICAN CHEMICAL SOCIETY Band 67, Nr. 10, Oktober 1945, Seiten 1711-1713; R.C. ELLINGSON et al.: "Pyrazine Chemistry. I. Derivatives of 3-Aminopyrazinoic Acid"<br>* Seite 1712, Spalte 1, Verbindung II * | 1 | |
| A | SYNTHESIS Nr. 11, November 1972, Seiten 628,630; P.K. KADABA et al.: "Esterification of Heterocyclic Carboxylic Acids Using a Boron Trifluoride Etherate-Alcohol Reagent"<br>* Seite 629, Tabelle * | 1 | |
| A,D | JUSTUS LIEBIGS ANNALEN DER CHEMIE Band 660, 1962, Seiten 98-103; F. DALLACKER et al.: "Darstellung und Reaktionen von 2-Amino-Pyrazin-Carbonsäure-(3)-Methylester"<br>* Seite 101, Verbindung I * | 1 | RECHERCHIERTE SACHGEBIETE (Int. Cl.5)<br><br>C 07 D 241/00 |
| A | DE-A-1 470 053 (MERCK & CO. INC.)<br>* Beispiele 16,18 * | 1 | |
| A | HOUBEN-WEYL: "METHODEN DER ORGANISCHEN CHEMIE" Band E 5, 1985, Georg Thieme Verlag, Stuttgart, DE; W. BAUER et al.: "Carbonsäuren und Carbonsäure-Derivate"<br>* Seiten 684-690 * | 1 | |

Der vorliegende Recherchenbericht wurde für alle Patentansprüche erstellt

| Recherchenort | Abschlußdatum der Recherche | Prüfer |
|---|---|---|
| Berlin | 12 November 90 | VAN AMSTERDAM L.J.P. |